# FASCICULE DE BREVET EUROPEEN

(11) **EP 0 814 716 B1**
(45) Date de publication et mention de la délivrance du brevet: **02.07.2003**
(21) Numéro de dépôt: 96905912.0
(22) Date de dépôt: 05.03.1996
(51) Int. Cl.: A61B 17/70

(54) **INSTRUMENTATION RACHIDIENNE NOTAMMENT POUR TIGE**
HILFSVORRICHTUNG FÜR DIE WIRBELSÄULENOSTEOSYNTHESE, INSBESONDERE ZUM GEBRAUCH MITVERBINDUNGSSTANGEN
SPINAL INSTRUMENTS, PARTICULARLY FOR A ROD

(30) Priorité: 06.03.1995 FR 9502580
(43) Date de publication de la demande: 07.01.1998
(73) Titulaire: Stryker France S.A., 93290 Tremblay-en-France (FR)
(72) Inventeur: PETRETO, Eric, F-33610 Cestas (FR)
(74) Mandataire: Le Forestier, Eric
(86) Numéro de dépôt international: FR9600342
(87) Numéro de publication internationale: WO96027340

(56) Documents cités:
- EP-A- 0 585 518
- FR-A- 2 682 280
- FR-A- 2 693 365
- US-A- 4 771 767
- US-A- 5 222 954
- US-A- 5 352 226

## Description

La présente invention est relative à une instrumentation rachidienne notamment pour tige d'union essentiellement lisse comprenant un élément d'ancrage osseux comportant une partie d'ancrage séparée d'une tête filetée saillante, associée à un écrou, par un corps intermédiaire de section polygonale, permettant le vissage dans le cas où la partie d'ancrage est une vis et formant butée en sa partie supérieure, et un ensemble de réception-blocage apte à recevoir ladite tige formé d'un élément formant pince, comprenant deux branches réunies par une zone de liaison, les branches étant pourvues d'orifices en vis-à-vis, au travers desquels peut être engagée la tête filetée jusqu'à la butée, et la pince délimitant entre les branches un passage.

Elle est également relative à un ensemble de réception-blocage destiné notamment à relier une tige à un élément d'ancrage.

Dans le brevet FR-2 657 775 est décrite notamment une instrumentation rachidienne à tige destinée à réduire et contenir le rachis, comprenant une vis pédiculaire à tête cylindrique filetée recevant une pièce engagée sur la tête filetée permettant le blocage d'une tige d'union entre une partie d'appui de ladite pièce et la partie conique d'un écrou de blocage engagé sur la tête filetée.

Ce système permet de bloquer la tige par rapport aux vis, mais la tige se trouve dans une orientation par rapport à la vis, à savoir orthogonale à la vis, qui est la même pour toutes les vis et ne peut être modifiée.

Dans le brevet français 2 693 365 on a décrit une instrumentation rachidienne à tige qui permet de régler dans une certaine mesure l'angle formé par les axes respectifs de la tige et de la vis dans un plan déterminé (c'est-à-dire sans que ce plan puisse pivoter d'un certain angle autour de l'axe de la tige) et de bloquer dans cette position la tige sur la vis.

Ce dispositif comprend une vis pédiculaire à tête filetée comportant un corps intermédiaire, un ensemble de réception-blocage engagé sur ladite tête filetée par un orifice oblong et muni d'un logement pour recevoir la tige latéralement à la vis et venant en butée sur la surface supérieure de forme sphérique du corps intermédiaire. La face inférieure tronconique de l'écrou vissé sur la tête filetée vient en contact en une zone opposée à la zone de contact avec la tige, avec une surface tronconique correspondante ménagée sur ledit ensemble de réception-blocage.

Il est néanmoins souhaitable de disposer d'un ensemble de réception-blocage permettant d'accroitre l'angulation de la tige par rapport à la vis, et donc de diminuer encore davantage le cintrage des tiges et en conséquence la tension de l'agencement réunissant les différentes vis pédiculaires, tout en améliorant notablement le blocage et donc la rigidité de l'ensemble.

Le document US 5 222 954 décrit par ailleurs une instrumentation rachidienne pour tige d'union conforme au préambule de la revendication 1 et comportant une partie d'ancrage, une tête filetée saillante, associée à un écrou, un corps intermédiaire et une pièce de réception-blocage 26 formée d'un orifice 28 permettant l'engagement de la tête filetée saillante 16 et d'un passage 34 dont l'axe est perpendiculaire à l'axe de l'orifice 28 destiné à recevoir la tige d'union 40. On remarquera que ce dispositif ne permet pas d'orienter la tige d'union dans toutes les directions, comme ceci est possible avec le dispositif selon la présente invention.

De même, les documents EP-A-585 518 et US 5 352 226 ne permettent pas l'orientation de la tige d'union dans toutes les directions.

Le document US 4 771 767 nécessite l'utilisation d'une tige filetée, ce qui présente l'inconvénient qu'elle ne soit pas modulable pour s'adapter aux déformations ou à l'anatome du rachis.

Le but de la présente invention est donc de proposer une instrumentation rachidienne présentant un blocage de la tige amélioré, tout en permettant d'orienter la tige d'union dans toutes les directions suivant une certaine angulation par rapport à l'axe neutre

L'invention est conforme à la revendication 1.

L'instrumentation rachidienne est constituée d'un matériau biocompatible utilisé pour l'implantation chirurgicale, notamment l'inox ou un alliage à base de titane.

De manière connue, les éléments d'ancrage osseux peuvent être des vis pédiculaires ou des crochets. D'autres moyens d'ancrage peuvent être utilisés dans le cadre de la présente invention.

Par "tige filetée", on entend un élément essentiellement lisse, c'est-à-dire qui peut coulisser au travers de la bague librement avant serrage.

La tige peut être un élément d'union lisse d'un diamètre suffisant pour résister aux contraintes et efforts du rachis, la surface lisse ayant l'avantage d'être modulable pour obtenir une certaine courbure s'adaptant aux déformations ou à l'anatomie du rachis. La tige peut également être constituée de torons cylindriques souples tressés.

Pour faciliter le travail du chirurgien, il est avantageux de limiter au repos l'angulation de l'axe de la bague par rapport à l'axe de la cavité. Cette variante de réalisation permet une insertion plus aisée de la tige car le passage est toujours accessible. Une solution avantageuse consiste à munir la bague d'une ou plusieurs butées limitant sa rotation.

Afin d'améliorer le blocage de la bague, il est avantageux de prévoir des moyens additionnels appropriés qui accentueront la pression exercée en certains points de la bague par la cavité. De préférence donc, la cavité est pourvue d'au moins une arête vive apte à solliciter la bague. Cela peut être notamment une rainure ménagée dans la cavité dégageant deux arêtes vives appuyant en position verrouillée sur la bague. La qualité du serrage peut être également améliorée en rendant l'état de surface de la cavité et de la bague au moins partiellement rugueux par sablage ou usinage.

Pour améliorer encore ce blocage, l'écrou présente une face inférieure convexe sphérique qui vient s'adapter à une zone concave sphérique située autour de l'orifice sur la branche correspondante.

Avantageusement, la bague est annulaire, de préférence sphérique et présente une face extérieure convexe dont le diamètre est compris entre celui d'une partie centrale en renfoncement et celui de parties latérales de la cavité.

De préférence encore, la compressibilité de la bague est assurée par une fente traversante. Une rainure symétriquement opposée à la fente peut encore améliorer la flexibilité.

D'autres caractéristiques et avantages ressortiront de la description qui va suivre d'un mode de réalisation du dispositif de l'invention, description donnée à titre d'exemple et en regard des dessins annexés dans lesquels :
- la figure 1 est une vue en perspective éclatée d'un dispositif conforme à l'invention,
- la figure 2 est une vue en perspective d'un dispositif conforme à l'invention, après assemblage,
- la figure 3 est une vue de dessus d'une partie du dispositif au repos,
- la figure 4 est une vue en coupe suivant la ligne III-III de la partie de la figure 3,
- la figure 5 est une vue en coupe suivant AA de la partie des figures 3 et 4,
- la figure 6 est une vue en bout d'une autre partie du dispositif,
- la figure 7 est une vue en demi-coupe selon CC de la partie de la figure 6,
- la figure 8 est une vue en coupe partielle de l'implant assemblé et verrouillé,
- la figure 9 est une vue de dessus d'une variante de la partie des figures 3 à 5, et
- la figure 10 est une vue partielle en coupe de la partie de la figure 9.

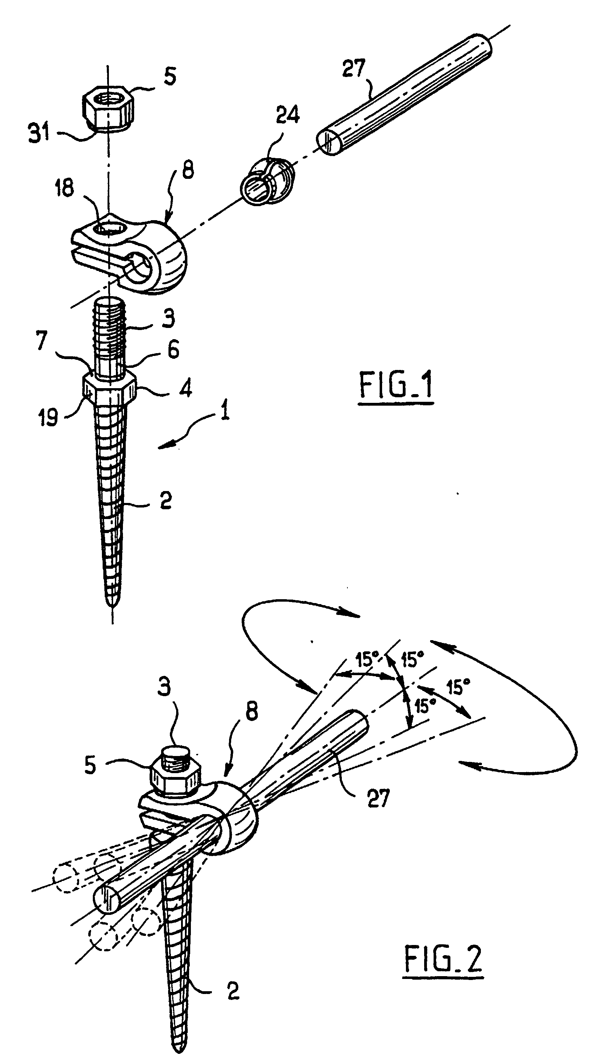

Selon la figure 1, une vis pédiculaire 1 comprend une partie inférieure ou pointe filetée 2, destinée à pénétrer dans un corps vertébral, et une partie supérieure ou tête filetée 3 sur laquelle peut être vissé un écrou 5. La pointe 2 et la tête 3 sont séparées par un corps intermédiaire 4, de section hexagonale dans le mode de réalisation illustré, permettant le vissage de la pointe 2 à l'aide d'une clef.

La tête filetée 3 est séparée du corps 4 par une partie lisse 6 dont le diamètre correspond par exemple au diamètre en fond de filet de la tête 3. Le corps hexagonal 4 forme une surface plane supérieure 7 formant butée.

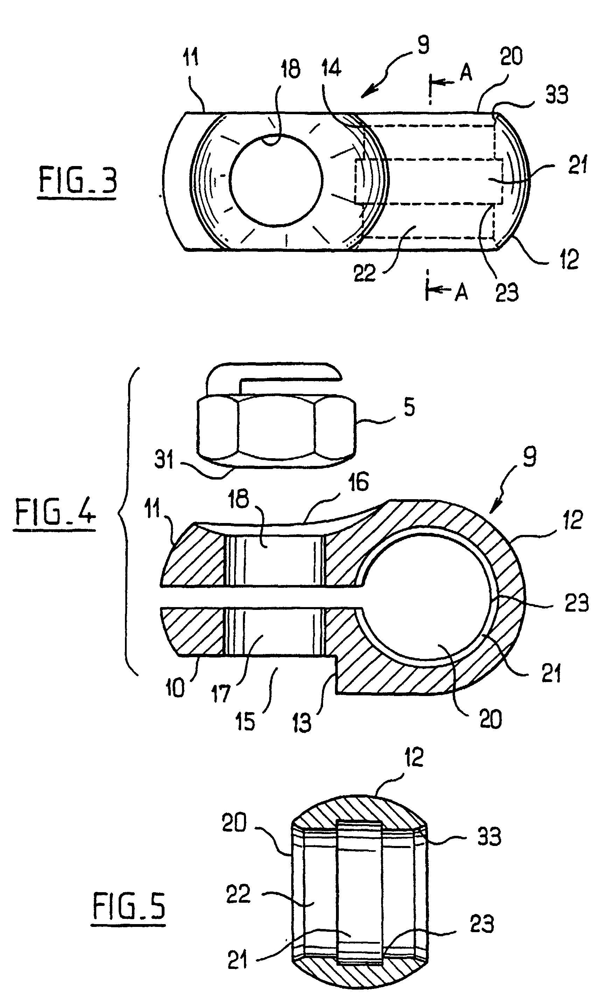

Aux figures 3, 4 et 5 est représentée une pièce formant pince 9 appartenant à un ensemble de réception-blocage 8 du dispositif. La pince 9 est formée de deux branches respectivement inférieure 10 et supérieure 11 et d'une zone de liaison 12 réunissant les branches. La face externe de la branche inférieure 10 présente un décrochement droit 13, délimitant une zone externe évidée inférieure 15. La face externe de la branche supérieure 11 présente une arête courbe 14 délimitant une zone évidée sphérique 16 concave centrée sur l'orifice 18. Les deux branches 10 et 11 sont pourvues de deux orifices coaxiaux 17 et 18 de diamètre approprié, en vis-à-vis pour permettre l'engagement de la tête filetée 3 jusqu'à la butée 7, une face verticale 19 du corps intermédiaire 4 venant alors en appui sur le décrochement inférieur 13 pour bloquer toute rotation relative de la vis pédiculaire 1 et de la pince 9. L'écrou 5 présente une face inférieure convexe sphérique 31 qui vient s'adapter à la zone convexe 16.

La pince délimite une cavité intérieure 20 ouverte sur les deux côtés de la pince. Ladite cavité est constituée d'une partie centrale cylindrique 21 flanquée de part et d'autre de deux parties latérales cylindriques 22 de rayon inférieur, les trois parties étant coaxiales. Les décrochements dus au rayon supérieur de la partie centrale dégagent deux arêtes vives circulaires 23. De préférence, le bord externe de chaque partie latérale cylindrique 23 présente un biseau 33 de façon à faciliter l'engagement de la tige et à ne pas limiter sa liberté de mouvement en angulation.

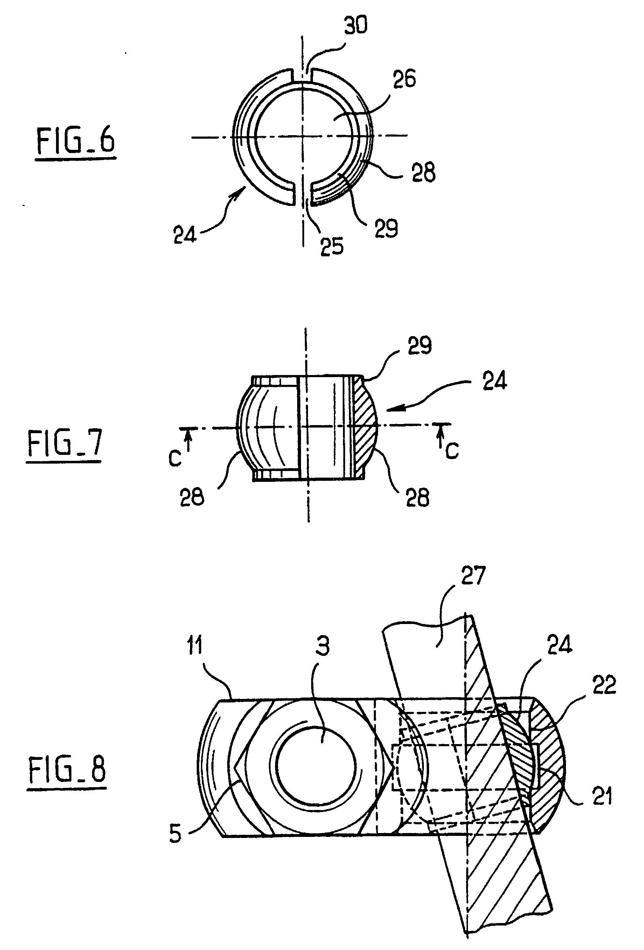

Selon les figures 6 à 8, à l'intérieur de la cavité est logée une bague 24, de longueur préférentiellement inférieure à celle de la cavité, munie d'une fente droite traversante 25. La bague 24 reçoit dans son passage intérieur 26 une tige d'union cylindrique 27. La face externe de la bague est pourvue d'un renflement 28 sphérique central dont le sommet est inséré en position non serrée de la pince dans la cavité délimitée par la partie centrale 21, ledit renflement étant prolongé de part et d'autre par deux parties extérieurement cylindriques 29 formant butées annulaires, de sorte que, en position non verrouillée, l'angulation de l'axe de la bague soit limitée par l'appui des butées annulaires sur les parois cylindriques 22 de la cavité. Une rainure 30, ménagée dans le renflement, symétrique à la fente 24, améliore la flexibilité de la bague.

On observera ici que le diamètre du renflement sphérique 28 de la bague est choisi supérieur au diamètre des parties 22 de la cavité, mais inférieur au diamètre de la partie centrale 21. De la sorte, la bague est, au repos, maintenue prisonnière avec jeu dans la cavité.

On observera en outre que la limitation du déplacement angulaire de la bague assurée par les butées 29 permet de garantir que le passage 26 de la bague soit correctement aligné avec l'accès de la cavité 20 de la pince.

L'angulation ainsi définie, c'est-à-dire l'angle de rotation possible de la tige autour de l'axe de la cavité cylindrique est de préférence d'environ 15°. Le vissage de l'écrou 5 sur la tête filetée serre les branches 10, 11 de la pince 9 ce qui vient comprimer la bague 24. Du fait de la flexibilité de la bague donnée par la fente 25 et la saignée 30, la bague vient à son tour pincer la tige d'union, qui se trouve fermement bloquée dans la position choisie par le praticien.

On observera ici que le fait que la pince 9 s'appuie sur la bague 24 par deux arêtes vives 23 assure un blocage d'une excellente qualité.

On pourra améliorer encore la qualité du blocage en rendant l'état de surface de la bague et de son passage et le cas échéant de la tige d'union au moins partiellement rugueux par exemple par sablage ou usinage.

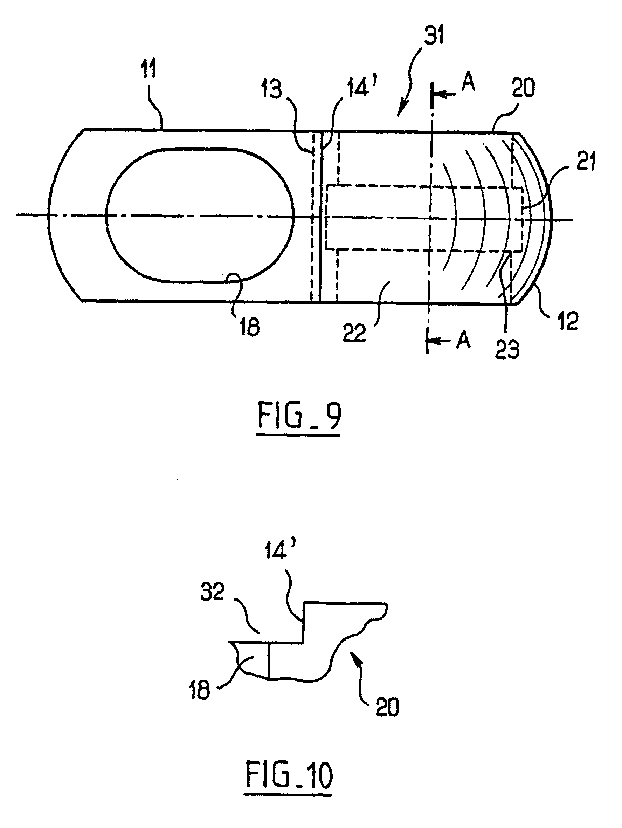

Selon une variante de la pièce des figures 3 à 5, représentée aux figures 9 et 10, les orifices 17,18 des branches 10,11 sont de forme oblongue dans le sens longitudinal des branches du dispositif. En outre, la face externe de la branche supérieure 11 présente un décrochement droit 14' délimitant une zone évidée 32. Un écrou plat (non représenté) vient se loger partiellement dans la zone évidée. Le décrochement 14' se situe à distance suffisante de l'écrou pour permettre son vissage à l'aide d'une clef.

Cette variante permet de faire varier l'entraxe (distance entre l'axe de la tige d'union et celui de la tête filetée), ce qui permet de s'adapter aux différents morphotypes rencontrés et de limiter, par coopération avec les possibilités d'orientation, le degré de cintrage des tiges.

Selon une autre variante non illustrée, la pince 9 est pourvue sur une partie de son étendue de deux fentes médianes inférieure et supérieure pour augmenter sa déformabilité élastique.

Enfin, l'invention n'est pas limitée aux modes de réalisation décrits ci-dessus mais en couvre au contraire toutes les variantes. Notamment, cette instrumentation rachidienne peut être associée à une instrumentation à plaque telle qu'elle est décrite dans le brevet FR-B-2 657 775.

## Revendications

1. Instrumentation rachidienne notamment pour tige d'union orientable essentiellement lisse, comprenant un élément d'ancrage osseux (1), ledit élément comportant une partie d'ancrage (2) séparée d'une tête filetée saillante (3), associée à un écrou (5), par un corps intermédiaire (4), et un ensemble (8) de réception-blocage d'une tête d'union formé d'un élément formant pince (9), comprenant deux branches (10, 11) réunies par une zone de liaison (12), les branches (10,11) étant pourvues d'orifices (17,18) en vis-à-vis, au travers desquels peut être engagée la tête filetée, et la pince délimitant entre les branches un passage (20); **caractérisée en ce que** le corps intermédaire est de section polygonale (19) et forme butée (7) en sa partie supérieure, la tête pouvant être engagée dans les orifices jusqu'à la butée (7), ledit passage définissant une cavité et **en ce qu'**il est prévu une bague compressible (24) logée dans la cavité et munie d'un passage central (25) pour recevoir la tige d'union, l'ensemble cavité-bague formant un montage à rotule, de sorte que le serrage des deux branches au moyen de l'écrou engagé sur la tête filetée provoque le blocage de la bague et de la tige d'union dans la position angulaire souhaitée.

2. Instrumentation rachidienne selon la revendication 1, **caractérisée en ce que** la cavité (20) est pourvue d'au moins une arête vive (23) apte à solliciter la bague (24) lors du serrage.

3. Instrumentation rachidienne selon l'une des revendications 1 à 2, **caractérisée en ce que** la cavité (20) est formée d'une partie centrale (21) cylindrique flanquée de part et d'autre de deux parties latérales (22) cylindriques coaxiales à ladite partie centrale et de rayon inférieur à celui de cette dernière de manière à dégager au niveau des décrochements deux arêtes vives circulaires (23).

4. Instrumentation rachidienne selon la revendication 3, **caractérisée en ce que** le bord externe de chaque partie latérale cylindrique (23) est biseauté (33).

5. Instrumentation rachidienne selon la revendication 3 ou 4, **caractérisée en ce que** la bague est annulaire et présente une face externe, convexe dont le diamètre est compris entre celui de la partie centrale (21) et celui des parties latérales (22) de la cavité (20).

6. Instrumentation rachidienne selon la revendication 5, **caractérisée en ce que** la face extérieure de la bague (20) est sphérique.

7. Instrumentation rachidienne selon l'une des revendications 1, 5 ou 6, **caractérisée en ce que** la bague est munie d'une fente traversante (25).

8. Instrumentation rachidienne selon la revendication 1, **caractérisée en ce qu'**au repos l'angulation de l'axe de la bague (24) par rapport à l'axe de la cavité est limitée.

9. Instrumentation rachidienne selon la revendication 8, **caractérisée en ce que** la face extérieure de la bague (24) est prolongée de part et d'autre par des parties cylindriques (29) formant butées de limitation de l'angulation.

10. Instrumentation rachidienne selon l'une des revendications 5 à 9, **caractérisée en ce que** la face extérieure convexe présente une rainure (30) symétrique à la fente (25) par rapport à l'axe de la bague.

11. Instrumentation rachidienne selon l'une des revendications 1 à 10, **caractérisée en ce que** la branche inférieure (10) présente un décrochement (13) contre lequel peut s'appuyer une face latérale du corps intermédiaire (4) de façon à bloquer la rotation relative de la vis pédiculaire et l'élément formant la pince.

12. Instrumentation rachidienne selon l'une des revendications 1 à 11, **caractérisée en ce que** la branche supérieure (11) présente au niveau de l'orifice (18) une zone concave sphérique (16) dans laquelle vient se loger la face inférieure (31) concave sphérique d'un écrou (5).

13. Instrumentation rachidienne selon l'une des revendications 1 à 11, **caractérisée en ce que** la branche supérieure (11) présente un décrochement (14') dégageant une zone évidée (32) permettant de loger au moins partiellement l'écrou.

14. Instrumentation rachidienne selon l'une des revendications précédentes, **caractérisée en ce que** la cavité et/ou la bague présentent au moins localement une surface rugueuse.

15. Instrumentation rachidienne selon l'une des revendications 1 à 14, **caractérisée en ce que** les orifices (17,18) sont de forme oblongue dans le sens longitudinal de façon à faire varier l'entraxe entre la tige d'union et la tête filetée (3).

16. Instrumentation rachidienne selon l'une quelconque des revendications 1 à 15, **caractérisée en ce que** ledit élément est d'une seule pièce.

## Patentansprüche

1. Instrumentelle Ausrüstung für Rückgrat, insbesondere für eine im wesentlichen glatte, ausrichtbare Verbindungsstange, mit einem Element zur Knochenverankerung (1), wobei dos Element einen Verankerungsbereich (2), welcher von einem vorspringenden, gewindeten, mit einer Mutter (5) verbundenen Kopf (3) durch einen Zwischenkörper getrennt ist, und eine Aufnahme-Blockier-Einheit (8) für einen Verbindungskopf aufweist, weiche gebildet ist aus einem eine Klemme (9) bildenden Element mit zwei Armen (10, 11), welche durch einen Verbindungsbereich (12) verbunden sind, wobei die Arme (10, 11) mit einander gegenüberliegenden Öffnungen (17, 18) versehen sind, durch welche der gewindete Kopf eingeschoben werden kann, und wobei die Klemme zwischen den Armen einen Durchgang (20) abgrenzt, **dadurch gekennzeichnet, dass** der Zwischenkörper einen polygonalen Querschnitt (19) aufweist und in seinem oberen Bereich einen Anschlag (7) bildet, wobei der Kopf bis zu dem Anschlag (7) in die Öffnungen eingegriffen erscheint, wobei der Durchgang einen Hohlraum definiert, und dass ein In dem Hohlraum gelogerter und mit einem zentralen Durchgang (25) zur Aufnahme der Verbindungsstange versehener, zusammendrückbarer Ring (24) vorgesehen ist, wobei die Hohlraum-Ring-Anordnung einen kugelgelenkarfigen Zusammenbau bildet, derart, dass das Festspannen der beiden Arme mittels der auf dem gewindeten Kopf eingegriffenen Mutter die Blockierung des Ringes und der Verbindungsslange in der gewünschten Winkelposilion bewirkt.

2. Instrumentelle Ausrüstung für Rückgrat gemäß Anspruch 1, **dadurch gekennzeichnet, dass** der Hohlraum (20) mit wenigstens einer scharfen Kante (23) versehen ist, welche in der Lage ist, den Ring (24) während des Festspannens zu beanspruchen,

3. Instrumentelle Ausrüstung für Rückgrat gemäß einem der Ansprüche 1 bis 2, **dadurch gekennzeichnet**, doss der Hohlraum (20) von einem zylindrischen Mittelbereich (21) gebildet ist, welcher beidseits von zwel zylindrischen Seitenbereichen (22) eingerahmt wird, welche koaxial zu dem Mittelbereich angeordnet sind und einen kleineren Radius als dieser letztere derart aufweisen, dass sie auf der Höhe der Absätze zwei kreisförmige scharfe Kanten (23) freilegen,

4. Instrumentelle Ausrüstung für Rückgrat gemäß Anspruch 3, **dadurch gekennzeichnet, dass** die Außenkonte jedes zylindrischen Seitenbereiches (23) geschliffen ist (33).

5. Instrumentelle Ausrüstung für Rückgrat gemäß Anspruch 3 oder 4, **dadurch gekennzeichnet, dass** der Ring ringförmig ist und eine konvexe Außenfläche aufweist, deren Durchmesser zwlschen demjenigen des Mittelbereiches (21) und demjenigen der Seitenbereiche (22) des Hohlraumes (20) liegt.

6. Instrumentelle Ausrüstung für Rückgrat gemäß Anspruch 5, **dadurch gekennzeichnet, dass** die Außenfläche des Ringes (24) kugelförmig ist.

7. Instrumentelle Ausrüstung für Rückgrat gemäß einem der Ansprüche 1, 5 oder 6, **dadurch gekennzeichnet**, doss der Ring mit einer durchquerenden Spalte (25) versehen ist,

8. Instrumentelle Ausrüstung für Rückgrat gemäß Anspruch 1, **dadurch gekennzeichnet, dass** in Ruhestellung die winkelige Anordnung der Achse des Ringes (24) bezüglich der Achse des Hohlraumes begrenzt ist.

9. Instrumentelle Ausrüstung für Rückgrat gemäß Anspruch 8, **dadurch gekennzeichnet, dass** die Außenfläche des Ringes (24) beidseits von zyllndrischen Bereichen (29) verlängert ist, welche Begrenzungsanschläge für die winkelige Anordnung bilden.

10. Instrumentelle Ausrüstung für Rückgrat gemäß einem der Ansprüche 5 bis 9, **dadurch gekennzeichnet**, doss die konvexe Außenfläche eine Rille (30) aufweist, welche symmetrisch zu der Spalte (25) bezüglich der Achse des Ringes verläuft.

11. Instrumentelle Ausrüstung für Rückgrat gemäß einem der Ansprüche 1 bis 10, **dadurch gekennzeichnet, dass** der untere Arm (10) einen Absatz (13) aufweist, gegen welchen sich eine Seitenfläche des Zwischenkörpers (4) derart lehnen konn, dass die relative Drehung der Stielschraube blockiert wird, und gegen welchen sich das die Klemme bildende Element lehnen kann.

12. Instrumentelle Ausrüstung für Rückgrat gemäß einem der Ansprüche 1 bis 11, **dadurch gekennzeichnet, dass** der obere Arm (11) auf der Höhe der Öffnung (18) einen kugelförmigen, konkaven Bereich (16) aufweist, in welchen sich die kugelförmige, konkave untere Fläche (31) einer Mutter (5) lagert.

13. Instrumentelle Ausrüstung für Rückgrat gemäß einem der Ansprüche 1 bis 11, **dadurch gekennzeichnet**, doss der obere Arm (11) einen Absatz (14') aufweist, welcher einen ausgehöhlten Bereich (32) freilegt, welcher ermöglicht, die Mutter wenigstens teilweise zu lagem.

14. Instrumentelle Ausrüstung für Rückgrat gemäß einem der vorherigen Ansprüche, **dadurch gekennzeichnet, dass** der Hohlraum und/oder der Ring wenigstens örtlich eine roue Oberfläche aufweisen.

15. Instrumentelle Ausrüstung für Rückgrat gemäß einem der Ansprüche 1 bis 14, **dadurch gekennzeichnet, dass** die Öffnungen (17, 18) im Längssinn eine längliche Form derart aufweisen, dass sie den Achsabstond zwischen der Verbindungsstange und dem gewindeten Kopf (3) varlieren lassen.

16. Instrumentelle Ausrüstung für Rückgrat gemäß irgendeinem der Ansprüche 1 bis 15, **dadurch gekennzeichnet**, doss das Element aus einem einzigen Stück besteht.

## Claims

1. Spinal instruments, especially for an essentially smooth adjustable connecting rod, comprising a bone anchor member (1), said member having an anchor portion (2) separated from a projecting screwthreaded head (3) associated with a nut (5) by an intermediate body (4), and a receiving and locking assembly (8) for a connecting head formed by a member forming a clamp (9) having two branches (10, 11) linked by a connecting area (12), the branches (10, 11) being provided with aligned orifices (17, 18) through which the screwthreaded head can be passed and the clamp delimiting a passage (20) between the branches, **characterized in that** the intermediate body is of polygonal section (19) and the upper portion forms an abutment (7), it being possible for the head to be passed into the orifices as far as the abutment (7), said passage defining a cavity and **in that** it comprises a compressible ring (24) housed in the cavity and having a central passage (25) to receive the connecting rod, the cavity-ring assembly forming a ball joint so that the clamping of the branches by means of the nut engaged on the screwthreaded head locks the ring and the connecting rod in the required angular position.

2. Spinal instruments according to claim 1 **characterized in that** the cavity (20) has at least one sharp edge (23) adapted to load the ring (24) during clamping.

3. Spinal instruments according to claim 1 or claim 2 **characterized in that** the cavity (20) has a cylindrical central portion (21) flanked on opposite sides by two cylindrical lateral portions (22) coaxial with said central portion and having a smaller radius than the latter so as to provide two circular sharp edges (23) at the steps.

4. Spinal instruments according to claim 3 **characterized in that** the external edge of each cylindrical lateral portion (23) is chamfered (33).

5. Spinal instruments according to claim 3 or claim 4 **characterized in that** the ring is annular and has a convex external face the diameter of which is between that of the central portion (21) and that of the lateral portions (22) of the cavity (20).

6. Spinal instruments according to claim 5 **characterized in that** the exterior face of the ring (20) is spherical.

7. Spinal instruments according to claim 1 or claim 5 or claim 6 **characterized in that** the ring has a through-slot (25).

8. Spinal instruments according to claim 1 **characterized in that** the angle of the axis of the ring (24) relative to the axis of the cavity at rest is limited.

9. Spinal instruments according to claim 8 **characterized in that** the exterior face of the ring (24) is extended on each side by cylindrical portions (29) forming angular movement limitation abutments.

10. Spinal instruments according to any of claims 5 to 9 **characterized in that** the convex exterior face has a groove (30) symmetrical to the slot (25) about the axis of the ring.

11. Spinal instruments according to any of claims 1 to 10 **characterized in that** the lower branch (10) has a step (13) against which a lateral face of the intermediate body (4) can bear to block relative rotation of the grub screw and the clamp member.

12. Spinal instruments according to any of claims 1 to 11 **characterized in that** the upper branch (11) has at the orifice (18) a spherical concave area (16) in which the spherical concave lower face (31) of a nut (5) is housed.

13. Spinal instruments according to any of claims 1 to 11 **characterized in that** the upper branch (11) has a step (14') producing a recessed area (32) housing the nut at least partially.

14. Spinal instruments according to any of the preceding claims **characterized in that** the cavity and/or the ring has a rough surface at least locally.

15. Spinal instruments according to any of claims 1 to 14 **characterized in that** the orifices (17, 18) are of oblong shape in the longitudinal direction so as to vary the distance between the axis of the connecting rod and the axis of the screwthreaded head (3).

16. Spinal instruments according to any of claims 1 to 15 **characterized in that** said member is made in one piece.
